# DEMANDE DE BREVET EUROPEEN

(11) **EP 4 501 341 A1**
(43) Date de publication de la demande: **05.02.2025**
(21) Numéro de dépôt: 24192183.2
(22) Date de dépôt: 31.07.2024
(51) Int. Cl.: A61K 36/185, A61P 43/00, A23L 33/00, A23L 33/105

(54) **PRÉPARATION DE COMBRETUM MICRANTHUM G. DON**

(30) Priorité: 03.08.2023 FR 2308423
(71) Demandeur: Larena, 49270 Oree D'Anjou (FR)
(72) Inventeur: Dubourdeaux, Michel, 03140 Taxat Senat (FR); BARDOT, Valérie, 03140 Target (FR); LEBLANC, Anne, 49110 Montrevault Sur Evre (FR)
(74) Mandataire: Jacobacci Coralis Harle

(57) **Abrégé**

La présente invention concerne une préparation de *Combretum micranthum* G. Don, pour son utilisation chez des sujets humains ou animaux, dans le traitement et/ou la prévention de la douleur.

La présente invention concerne encore une composition, pharmaceutique ou non-pharmaceutique, contenant une préparation de *Combretum micranthum* G. Don selon l'invention, pour son utilisation chez des sujets humains ou animaux, dans le traitement et/ou la prévention de la douleur.

## Description

### Domaine technique de l'invention

La présente invention concerne le domaine technique des préparations de plantes, en particulier les extraits de plantes, pour leur utilisation chez des sujets humains ou animaux, dans le traitement et/ou la prévention de la douleur.

### Etat de la technique

Actuellement, la prise en charge médicamenteuse des états douloureux chroniques présente des limites.

Le mésusage des médicaments liés à la douleur est un problème très important. Ces mésusages concernent les antalgiques les plus référencés tels que le paracétamol, l'aspirine, les anti-inflammatoires ou la morphine et ses dérivés.

Les antalgiques sont considérés comme actifs et efficaces contre les douleurs aiguës mais présentent des effets secondaires significatifs s'ils sont utilisés de façon prolongée : troubles gastriques, rénaux, dépendance, accoutumance, etc.

Ainsi ces médicaments ne sont pas dénués d'effets indésirables et ne sont donc pas adaptés à une prise chronique ou à la prise en charge des douleurs chroniques.

Pour ces dernières, notamment les douleurs de type neuropathiques, les traitements sont des anti-dépresseurs et des antiépileptiques, qui ont moins d'effets indésirables mais avec une efficacité observable chez seulement 50% des patients.

Il existe ainsi un besoin de développer des traitements efficaces, sûrs et bien tolérés pour les patients.

Dans ce contexte, beaucoup de consommateurs se tournent vers des approches naturelles à base de plantes ou de préparations de plantes pour améliorer leur qualité de vie.

Les préparations de plantes offrent en effet un potentiel pour combler ce besoin en raison de leur efficacité, de leur faible toxicité et de leur disponibilité.

En conséquence, il y a un intérêt continu pour développer de nouvelles préparations de plantes adaptées au traitement et à la prévention de la douleur.

### Présentation de l'invention

Afin de remédier à l'inconvénient précité de l'état de la technique, la présente invention propose une préparation de *Combretum micranthum* G. Don, pour son utilisation chez des sujets humains ou animaux, dans le traitement et/ou la prévention de la douleur.

En effet, de manière surprenante, la présente invention propose un ingrédient végétal, issu de *Combretum micranthum* G Don, pour traiter ou prévenir la douleur chez les sujets humains ou animaux, avec un profil d'efficacité intéressant.

De plus, cet ingrédient végétal selon l'invention est avantageusement standardisé en termes de composition phytochimique et d'activité biologique.

Un tel ingrédient végétal constitue en outre une ressource naturelle composée d'un mélange de molécules actives (*versus* une molécule synthétique seule), adaptée à la prise chronique.

D'autres caractéristiques non limitatives et avantageuses du produit/procédé conforme à l'invention, prises individuellement ou selon toutes les combinaisons techniquement possibles, sont les suivantes :
- ladite douleur est choisie parmi les douleurs articulaires, viscérales, pelviennes, post-opératoires, inflammatoires, neuropathiques, arthritiques ;
- le traitement et/ou la prévention de la douleur est obtenu par l'intermédiaire d'un effet anti-inflammatoire ;
- le traitement et/ou la prévention de la douleur englobe une diminution de son intensité et/ou une diminution de sa durée ;
- la douleur est choisie parmi les douleurs légères, les douleurs modérées, et les douleurs sévères ;
- ladite préparation est issue d'une extraction à partir des feuilles ;
- ladite préparation contient des kinkeloïdes, à savoir avantageusement choisis parmi les kinkeloïdes A, les kinkeloïdes B, les kinkeloïdes C et les kinkeloïdes D.

Encore de manière générale, la préparation de *Combretum micranthum* G. Don selon l'invention consiste de préférence en une préparation issu d'un procédé choisi parmi les procédés suivants :
- un procédé d'extraction méthanolique comprenant les étapes successives suivantes :
   -- une étape de broyage du matériel végétal,
   -- une étape de macération du matériel végétal broyé dans un alcool, par exemple du méthanol,
   -- une étape de filtration des macérats,
   -- une étape de rassemblement des filtrats et évaporation pour éliminer l'alcool et l'eau,
   -- une étape de lyophilisation ;
- un procédé de préparation d'une préparation végétale, à partir de matière végétale, comprenant :
   -- une étape de contact entre la matière végétale et un solvant,
   -- au moins une étape d'extraction en phase liquide des principes actifs, réalisée sous chauffage et sous pression réduite, pour obtenir une fraction d'extraction contenant lesdits principes actifs et une matière végétale résiduelle, et
   -- une étape de séchage de ladite matière végétale résiduelle en présence de ladite fraction d'extraction, afin de permettre la fixation des principes actifs sur ladite matière végétale résiduelle,
- un procédé de préparation d'une préparation végétale comprenant :
   -- une étape de trempage d'une matière végétale broyée, en présence d'un solvant alcoolique, par exemple de l'éthanol,
   -- une étape de percolation par l'ajout d'un solvant alcoolique, par exemple de l'éthanol,
   -- une étape de retrempage par ajout d'un solvant alcoolique, par exemple de l'éthanol,
   -- une étape de filtration du solvant de retrempage,
   -- une étape de concentration du filtrat et lyophilisation,
- un procédé de préparation d'une préparation végétale comprenant :
   -- une étape de trempage de la matière végétale broyée, en présence d'un solvant alcoolique, par exemple de l'éthanol,
   -- une étape de percolation par l'ajout d'un solvant alcoolique, par exemple de l'éthanol,
   -- une étape de retrempage par ajout d'un solvant alcoolique, par exemple de l'éthanol,
   -- une étape de filtration du solvant de retrempage,
   -- une étape de concentration du filtrat par évaporation,
   -- une étape de stabilisation de la préparation sec à l'alcool,
   -- une étape de concentration de la préparation végétale, et
   -- une étape de stabilisation à la glycérine de la préparation.

La présente invention englobe également les procédés précités, pour l'obtention de la préparation de *Combretum micranthum* G. Don selon l'invention.

La présente invention concerne encore une composition contenant une préparation de *Combretum micranthum* G. Don selon l'invention, pour son utilisation chez des sujets humains ou animaux, dans le traitement et/ou la prévention de la douleur.

Ladite composition contient avantageusement en outre l'un au moins des ingrédients suivants : une préparation d'algue, une préparation de plante et des probiotiques, des vitamines, des minéraux ou tout autre ingrédient nutritionnel.

Cette composition est avantageusement adaptée à une utilisation dans l'industrie pharmaceutique, cosmétologique, agro-alimentaire, de la nutrition, des compléments alimentaires, et de la nutrition animale.

La présente invention concerne encore une utilisation non-thérapeutique d'une préparation de *Combretum micranthum* G. Don selon l'invention, pour son utilisation chez des sujets humains ou animaux, dans le traitement et/ou la prévention de la douleur.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée.

La présente invention concerne donc, de manière générale, les préparations (et en particulier les extraits) de *Combretum micranthum* G. Don, pour leur utilisation chez des sujets humains ou animaux, dans le traitement et/ou la prévention de la douleur.

Par « sujets humains », on entend en particulier les patients souffrant de douleurs chroniques ou aiguës. Les sujets humains peuvent également inclure des personnes en bonne santé qui souhaitent prévenir les douleurs liées à leur travail, à leur mode de vie, à l'âge, ou les personnes ayant subi une intervention médicale (chirurgicale ou dentaire par exemple) et souffrant de douleurs post-opératoires.

Par « sujets animaux », on entend en particulier les animaux de compagnie tels que les chiens et les chats, qui peuvent souffrir de douleurs chroniques liées à des maladies articulaires ou à d'autres affections, ainsi que les animaux d'élevage tels que les bovins, les porcins, les ovins et les équidés.

Le terme « traitement de la douleur » se réfère à une utilisation pour réduire, soulager ou éliminer la douleur.

Le terme « prévention de la douleur » se réfère à une utilisation pour prévenir ou réduire la douleur avant qu'elle ne se produise.

### Combretum micranthum G. Don

*Combretum micranthum* ou *Combretum micranthum* G. Don est une espèce d'arbuste non domestiquée que l'on trouve dans la jungle d'Afrique de l'Ouest.

Elle est aussi désignée *Bureava crotonoides, Combretum altum, Combretum floribundum, Combretum parviflorum, Combretum raimbaultii* ou encore kinkéliba (nom vernaculaire).

C'est un arbuste dense ou une liane, que l'on trouve couramment dans les champs cultivés et en jachère, principalement situés en Afrique subsaharienne, avec des rendements plus élevés au Sénégal, au Mali et au Burkina Faso.

Dans plusieurs pays de la savane tropicale d'Afrique de l'Ouest, les feuilles de *Combretum micranthum* sont utilisées comme une tisane traditionnelle populaire, en particulier pour ses propriétés diurétiques.

Encore de manière générale, *Combretum micranthum* G. Don est encore défini dans la Monographie de la pharmacopée Française, édition 1993.

### Préparation de Combretum micranthum G. Don et ses procédés d'extraction

Par « préparation de plante », on englobe avantageusement les préparations obtenues à partir des matières premières végétales, notamment en les réduisant en poudre ou en les traitant par un procédé d'extraction, de distillation, d'expression, de fractionnement, de purification, de concentration ou de fermentation.

Par « préparation de plante », on englobe en particulier les extraits de *Combretum micranthum* G. Don.

De préférence, la préparation de *Combretum micranthum* G. Don est issue d'un procédé d'extraction à partir des feuilles de *Combretum micranthum* G. Don.

Les feuilles de *Combretum micranthum* G. Don sont avantageusement des feuilles fraîches ou des feuilles séchées.

De manière générale, différents procédés d'extraction sont adaptés à l'obtention d'une préparation de *Combretum micranthum* G. Don selon l'invention.

Selon un premier mode de réalisation, le procédé d'extraction consiste en un procédé d'extraction méthanolique comprenant les étapes successives suivantes :
- une étape de broyage du matériel végétal,
- une étape de macération du matériel végétal broyé dans un alcool, par exemple du méthanol,
- une étape de filtration des macérats,
- une étape de rassemblement des filtrats et évaporation pour éliminer l'alcool et l'eau,
- une étape de lyophilisation.

Un tel procédé est encore développé dans le document suivant : M. Gainche et al., Anti-inflammatory and cytotoxic potential of new phenanthrenoids from Luzula sylvatica, Molecules, 2020, 25 (10), 2372).

Selon un deuxième mode de réalisation, le procédé d'extraction consiste en un procédé de préparation d'une préparation végétale, à partir de matière végétale, comprenant :
- une étape de contact entre la matière végétale et un solvant,
- au moins une étape d'extraction en phase liquide des principes actifs, réalisée sous chauffage et sous pression réduite, pour obtenir une fraction d'extraction contenant lesdits principes actifs et une matière végétale résiduelle, et
- une étape de séchage de ladite matière végétale résiduelle en présence de ladite fraction d'extraction, afin de permettre la fixation des principes actifs sur ladite matière végétale résiduelle.

Un tel procédé est par exemple décrit dans le document EP-2 080 436.

Selon un troisième mode de réalisation, le procédé d'extraction consiste en un procédé de préparation d'une préparation végétale comprenant :
- une étape de trempage d'une matière végétale broyée, en présence d'un solvant alcoolique, par exemple de l'éthanol,
- une étape de percolation par l'ajout d'un solvant alcoolique, par exemple de l'éthanol,
- une étape de retrempage par ajout d'un solvant alcoolique, par exemple de l'éthanol,
- une étape de filtration du solvant de retrempage,
- une étape de concentration du filtrat et lyophilisation.

Selon un quatrième mode de réalisation, le procédé d'extraction consiste en un procédé de préparation d'une préparation végétale comprenant :
- une étape de trempage de la matière végétale broyée, en présence d'un solvant alcoolique, par exemple de l'éthanol,
- une étape de percolation par l'ajout d'un solvant alcoolique, par exemple de l'éthanol,
- une étape de retrempage par ajout d'un solvant alcoolique, par exemple de l'éthanol,
- une étape de filtration du solvant de retrempage,
- une étape de concentration du filtrat par évaporation,
- une étape de stabilisation de la préparation sec à l'alcool,
- une étape de concentration de la préparation végétale, et
- une étape de stabilisation à la glycérine de la préparation.

Encore de manière générale, la préparation de *Combretum micranthum* G. Don selon l'invention contient des kinkeloïdes, composés de la famille des piperidine-flavan alkaloids.

Les kinkeloïdes sont par exemple décrits dans le document Jing Zhen et al., Total Synthesis of Novel Skeleton Flavan-Alkaloids, Molecules 2020, 25(19), 4491.

Par « kinkeloïdes », on englobe en particulier les kinkeloïdes A, les kinkeloïdes B, les kinkeloïdes C et les kinkeloïdes D.

Par kinkeloïdes A, on entend les composés chimiques présentant la formule chimique 1 suivantes : 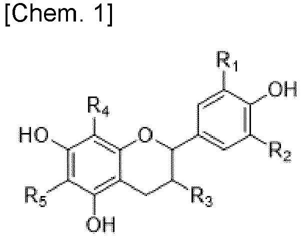 dans laquelle
R₁ est H,
R₂ est H,
R₃ est H,
et dans laquelle :
R₄ est H et R₅ est pipéridine, ou
R₄ est pipéridine et R₅ est H.

Par kinkeloïdes B, on entend les composés chimiques présentant la formule chimique 2 suivantes : 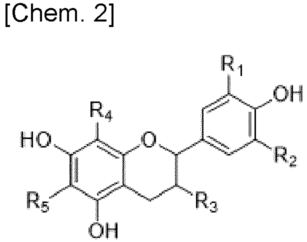 dans laquelle
R₁ est OH,
R₂ est H,
R₃ est H, et
dans laquelle
R₄ est H et R₅ est pipéridine, ou
R₄ est pipéridine et R₅ est H.

Par kinkeloïdes C, on entend les composés chimiques présentant la formule chimique 3 suivantes : 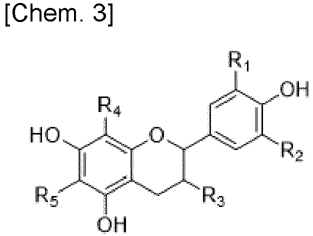 dans laquelle
R₁ est OH
R₂ est OH
R₃ est H,
dans laquelle
R₄ est H et R₅ est pipéridine, ou
R₄ est pipéridine et R₅ est H.

Par kinkeloïdes D, on entend les composés chimiques présentant la formule chimique 4 suivantes : 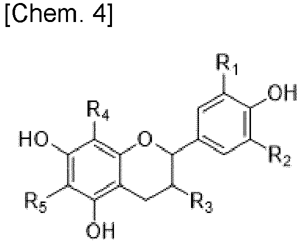 dans laquelle
R₁ est OH
R₂ est OH
R₃ est OH,
dans laquelle
R₄ est H et R₅ est pipéridine, ou
R₄ est pipéridine et R₅ est H.

De préférence, la préparation selon l'invention contient des kinkéloïdes B qui représentent de 70 à 90% des kinkéloïdes totaux.

Sans être limitatif, une posologie préférée pourrait être de 20 mg à 5 g de la préparation selon l'invention, par jour, en une ou plusieurs prises.

### Traitement et/ou prévention de la douleur

Par « douleur », on entend en particulier les douleurs articulaires, viscérales, pelviennes, post-opératoires, inflammatoires, neuropathiques, arthritiques.

Le terme « douleur articulaire » se réfère à une douleur ou une gêne ressentie dans une ou plusieurs articulations du corps. Plus particulièrement, l'invention vise à fournir une solution pour le traitement et/ou la prévention des douleurs articulaires, qui peuvent être causées par une variété de facteurs tels que des blessures, des inflammations, des maladies, ou une usure normale liée à l'âge. Les douleurs articulaires peuvent affecter n'importe quelle partie du corps, y compris les mains, les poignets, les coudes, les épaules, les hanches, les genoux, les chevilles et les pieds. Les symptômes peuvent inclure une douleur constante ou intermittente, une raideur, une enflure, une rougeur ou une chaleur autour de l'articulation affectée, ainsi qu'une perte de mobilité et de flexibilité.

Le terme « douleur viscérale » ou « douleur pelvienne » se réfère à une douleur ou une gêne ressentie dans les organes internes du corps, tels que l'estomac, les intestins, la vésicule biliaire, le foie, le pancréas, les reins ou la partie inférieure de l'abdomen. Les douleurs viscérales ou pelviennes peuvent être causées par des affections telles que des ulcères, des infections, des inflammations, des calculs ou des troubles fonctionnels.

Le terme « douleur post-opératoire » se réfère à la douleur et à l'inconfort qui surviennent après une intervention chirurgicale. La douleur post-opératoire est un phénomène courant qui peut varier en intensité et/ou en durée en fonction du type d'intervention, de la sensibilité individuelle et d'autres facteurs. La douleur post-opératoire peut être localisée au site de l'incision ou peut être ressentie dans d'autres parties du corps.

Les douleurs inflammatoires, associées à des phénomènes d'inflammation qui perdurent anormalement, englobent en particulier les douleurs articulaires. Dans ce cas, l'activation chronique des fibres de la douleur entraîne leur sensibilisation qui se généralise ensuite à tout le système de la douleur. Aussi, même en traitant la cause en périphérie, le système peut rester hyper réactif.

Les douleurs neuropathiques sont généralement liées à des atteintes du système nerveux central ou périphérique (lésions de nerfs, blessure, etc.), de la moelle épinière, liées aux amputations ou à un accident vasculaire cérébral. Ces lésions concernent directement le système de détection de la douleur : elles rendent le système d'alarme défaillant et incontrôlable par les antalgiques classiques.

Le terme « douleur arthritique » se réfère à la douleur et à l'inflammation qui surviennent dans les articulations affectées par l'arthrite. L'arthrite est une maladie qui se caractérise par l'inflammation des articulations, qui peut entraîner des douleurs, des raideurs, une réduction de la mobilité articulaire et d'autres symptômes. Les douleurs arthritiques peuvent varier en intensité, en durée et en fréquence en fonction de la gravité de la maladie et de la sensibilité individuelle.

De préférence, la préparation de *Combretum micranthum* G. Don assure le traitement et/ou la prévention de la douleur par l'intermédiaire d'un effet anti-inflammatoire.

De manière générale, par « traitement et/ou prévention de la douleur », la présente invention englobe une diminution de son intensité et/ou de sa durée.

Par « intensité » de la douleur, on englobe la douleur mesurée par des outils pour la caractériser et l'évaluer.

De manière générale, des questionnaires et des échelles de douleur permettent d'en décrire les manifestations, et d'en mesurer l'intensité ainsi que l'impact sur la qualité de vie.

Pour les adultes, l'échelle la plus utilisée est l'échelle numérique ou l'échelle visuelle analogique, graduée de 0 pour une absence de douleur, à 10 pour la douleur maximale imaginable.

Pour les enfants, les médecins utilisent souvent une échelle avec des visages.

Concernant les douleurs neuropathiques, deux échelles permettent respectivement de diagnostiquer ce type de douleurs (DN4) et d'évaluer leur intensité (NPSI).

De manière générale, la présente invention englobe une diminution de l'intensité et/ou de la durée pour la douleur mesurée chez un sujet recevant la préparation de *Combretum micranthum* G. Don selon l'invention par rapport à l'intensité et/ou à la durée pour la douleur mesurée chez un sujet ne recevant pas la préparation de *Combretum micranthum* G. Don selon l'invention.

La douleur englobe en particulier :
- les douleurs légères,
- les douleurs modérées, et
- les douleurs sévères.

Les douleurs légères sont des douleurs peu intenses qui peuvent être facilement supportées par le sujet et n'affectent pas ou peu son activité quotidienne.

Les douleurs modérées sont plus intenses et peuvent affecter le quotidien du sujet, en limitant par exemple ses mouvements ou sa capacité à travailler.

Les douleurs sévères sont les douleurs les plus intenses, qui peuvent être invalidantes et empêcher le sujet de mener une vie normale.

### Composition

La présente invention concerne encore les compositions contenant une préparation de *Combretum micranthum* G. Don selon l'invention, pour son utilisation chez des sujets humains ou animaux, dans le traitement et/ou la prévention de la douleur.

Une telle composition contient avantageusement en outre l'un au moins des ingrédients suivants :
- une préparation d'algue,
- une préparation de plante et
- des probiotiques, y compris des postbiotiques.

La préparation d'algue peut être choisi parmi différentes espèces d'algues, telles que le porphyra, une algue rouge également connue sous le nom de nori, la spiruline, la chlorelle ou une algue brune.

De préférence, la composition selon l'invention comprend une combinaison suivante :
- préparation de *Combretum micranthum* G. Don selon l'invention, et
- préparation de nori.

La préparation de plante peut provenir de différentes plantes, telles que le boswellia, le curcuma, les feuilles de cassis, la saule, la reine des prés, la prêle, l'harpagophytum, la scrofulaire, le cresson du Para (*Acmella oleracea*) ou le gingembre.

De préférence, la composition selon l'invention comprend une combinaison suivante :
- préparation de *Combretum micranthum* G. Don selon l'invention, et
- préparation de gingembre.

De manière générale, par « souche probiotique », on englobe en particulier les microorganismes vivants qui, lorsqu'ils sont ingérés en quantité suffisante, exercent des effets positifs sur la santé, au-delà des effets nutritionnels traditionnels.

La souche probiotique convenant à l'invention est physiologiquement acceptable. En d'autres termes, cette souche probiotique peut être administrée sans risques à l'animal ou l'homme.

Les souches probiotiques englobent notamment les genres lactobacilles (bactéries du genre *Lactobacillus*)*,* bifidobactéries (bactéries du genre *Bifidobacterium*), streptocoques (bactéries du genre *Streptococcus*) ou lactocoques.

Encore de manière générale, des exemples spécifiques de microorganismes probiotiques sont *Bifidobacterium adolescentis, Bifidobacterium animalis, Bifidobacterium bifidum, Bifidobacterium breve, Bifidobacterium lactis, Bifidobacterium longum, Bifidobacterium infantis, Bifidobacterium pseudocatenulatum, Lactobacillus acidophilus, Lactobacillus amylovorus, Lactobacillus casei (Shirota), Lactobacillus rhamnosus (souche GG), Lactobacillus brevis, Lactobacillus crispatus, Lactobacillus delbrueckii (subsp bulgaricus, lactis), Lactobacillus fermentum, Lactobacillus helveticus, Lactobacillus gallinarum, Lactobacillus gasseri, Lactobacillus johnsonii, Lactobacillus paracasei, Lactobacillus plantarum, Lactobacillus reuteri, Lactobacillus salivarius, Lactobacillus alimentarius, Lactobacillus curvatus, Lactobacillus casei subsp. casei, Lactobacillus sake Lactococcus lactis, Enterococcus (faecalis, faecium), Lactococcus lactis (subspp lactis ou cremoris), Leuconstoc mesenteroides subsp dextranicum, Pediococcus acidilactici, Sporolactobacillus inulinus, Streptococcus salvarius subsp. Thermophilus, Streptococcus thermophilus, Staphylococccus camosus, Staphylococcus xylosus, Saccharomyces (cerevisiae* ou encore *boulardii), Bacillus (cereus var toyo ou subtilis), Bacillus coagulans, Bacillus licheniformis, Escherichia coli strain nissle, Propionibacterium freudenreichii* et leurs mélanges.

Plus particulièrement, il s'agit de microorganismes probiotiques issus du groupe des bactéries lactiques, comme notamment les *Lactobacillus* et/ou les *Bifidobacterium.* A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei ou Bifidobacterium bifidum, Bifidobacterium breve, Bifidobacterium longum, Bifidobacterium animalis, Bifidobacterium lactis, Bifidobacterium infantis, Bifidobacterium adolescentis ou Bifidobacterium pseudocatenulatum* et leurs mélanges.

De manière générale, ladite au moins une souche probiotique est avantageusement choisie parmi les souches probiotiques vivantes ou les souches probiotiques inactivées.

Par forme « vivante », on entend une forme dotée de la capacité à se multiplier sous réserve de la placer dans un environnement propice à la récupération de cette capacité.

Ainsi, au sens de la présente invention, le terme vivant couvre l'état dit dormance dans lequel les microorganismes peuvent être placés suite à un traitement physicochimique tel que par exemple la lyophilisation.

De manière générale, la souche probiotique est avantageusement mise en oeuvre sous une forme lyophilisée.

Ce type de formulation possède les avantages d'être facilement accessible, de mise en oeuvre aisée et de ne soulever aucune difficulté et/ou contrainte en termes de stockage. Par ailleurs, il est compatible avec le conditionnement de microorganismes à l'état de dormance.

Cette lyophilisation peut être réalisée selon des méthodes conventionnelles ou selon une méthode décrite par exemple dans le document FR-3 103 827.

Par ailleurs, le terme « inactivé » désigne des souches ayant subi un traitement visant à les tuer. De tels traitements peuvent consister, à titre d'exemple non limitatif, en un traitement en autoclave, par ultra-sons, une homogénéisation haute pression ou encore un choc osmotique.

Les souches inactivées sont avantageusement intactes.

La souche probiotique est avantageusement inactivée par la chaleur, dite encore « souche probiotique tyndallisée ».

La concentration en souche probiotique, dans la composition, est par exemple de 10⁷ à 10¹² UFC par dose quotidienne.

La concentration en souche probiotique dans la composition peut être d'une manière générale une quantité efficace permettant d'obtenir l'effet recherché. Cette quantité peut être déterminée par la personne du métier au moyen de ses connaissances générales et par des tests usuels. Cette quantité peut par exemple être, pour une application topique, en dose journalière exprimée en CFU, d'environ 10⁶ à environ 10¹², de préférence au moins 10⁶ à environ 10¹⁰, de préférence d'environ 10⁸ à environ 10⁹. Elle peut être alternativement d'environ 10⁵ à environ 10¹² organismes/g de produit.

La souche probiotique peut encore être incluse à hauteur de de 0,01% à 50% dans la composition, par exemple entre 0,01% et 40%, ou entre 0,01% et 30%, ou entre 0,01% et 10%, ou entre 0,01% et 5%, ou entre 0,01% et 1%, par exemple environ 0,3% (poids/poids).

La composition peut également contenir des postbiotiques, correspondent à des préparations de micro-organismes, avantageusement choisis parmi les souches probiotiques précitées, inanimés et/ou de leurs composants conférant un bénéfice pour la santé de l'hôte.

La composition peut encore contenir d'autres types d'ingrédients, par exemple des vitamines, des minéraux ou tout autre ingrédient nutritionnel.

Plus particulièrement, la composition peut contenir des ingrédients nutraceutiques comme la vitamine C, la glucosamine, la chondroïtine, le palmitoyléthanolamide ou des acides gras.

La composition selon l'invention contient avantageusement une combinaison d'ingrédients adaptés à une utilisation dans l'industrie pharmaceutique, cosmétologique, agro-alimentaire, de la nutrition (y compris les denrées alimentaires destinées à des fins médicales spéciales - DADFMS), des compléments alimentaires, de la nutrition animale.

La composition selon l'invention comprend ainsi avantageusement :
- une préparation de *Combretum micranthum* G. Don selon l'invention, pour son utilisation chez des sujets humains ou animaux, adapté à un traitement et/ou la prévention de la douleur, et
- au moins un support ou un excipient, acceptable en fonction du l'utilisation envisagée.

La préparation de *Combretum micranthum* G. Don de la présente invention peut être préparé sous la forme d'une composition pharmaceutique, qui peut être une formulation telle que des comprimés, des capsules, des poudres, des granules, des solutions, des pastilles, des gelées, des préparations de crème, des sirops, des suspensions, des teintures, des aérosols et autres.

La préparation de *Combretum micranthum* G. Don de la présente invention peut également être préparé sous la forme d'une composition non-pharmaceutique.

La composition non-pharmaceutique englobe les compositions cosmétologiques, les compositions agro-alimentaires, les compositions nutritionnelles, les compléments alimentaires, les compositions pour la nutrition animale.

La composition non-pharmaceutique peut être préparée par des techniques de préparation généralement connues, et des additifs acceptables peuvent être ajoutés à la composition non-pharmaceutique.

Par exemple, la composition non-pharmaceutique selon l'invention consiste avantageusement en un complément alimentaire.

Par « complément alimentaire », on entend avantageusement les compléments alimentaires qui sont soumis à l'ensemble des dispositions générales du droit alimentaire mais aussi aux règles spécifiques définies par la directive 2002/46/CE du Parlement européen et du Conseil du 10 juin 2002 relative au rapprochement des législations des États membres concernant les compléments alimentaires, transposée en droit français par le décret n°2006-352.

Par « compléments alimentaires », on englobe avantageusement les denrées alimentaires dont le but est de compléter le régime alimentaire normal et qui constituent une source concentrée de nutriments ou d'autres substances ayant un effet nutritionnel ou physiologique seuls ou combinés, commercialisés sous forme de doses, à savoir les formes de présentation telles que les ampoules de liquide, les flacons munis d'un compte-gouttes et les autres formes analogues de préparations liquides.

De manière générale, la composition selon l'invention est adaptée à une administration par voie orale, per os ou topique.

### Utilisation non-thérapeutique

La présente invention englobe encore une utilisation non-thérapeutique d'une préparation de *Combretum micranthum* G. Don selon l'invention, pour son utilisation chez des sujets humains ou animaux, dans le traitement et/ou la prévention de la douleur.

Bien entendu, diverses autres modifications peuvent être apportées à l'invention dans le cadre des revendications annexées.

### Exemples

### Matériel

- Nom latin : *Combretum micranthum* G.Don
- Partie de plantes : feuilles
- Etat de la plante : sec

### Exemple 1 - procédé d'extraction selon le premier mode de réalisation

### 1. Protocole d'extraction

- broyer le matériel végétal
- faire macérer les échantillons dans du méthanol (10% w/v) 3 x 24h sous agitation mécanique constante
- filtrer les échantillons chaque 24h
- rassembler les filtrats après chaque extraction, évaporer les filtrats pour éliminer le méthanol
- lyophiliser.

Le rendement d'extraction en matière sèche : 20 à 35%
- fractionner la préparation méthanol selon le protocole développé par Gainche et al. (M. Gainche et al., Anti-inflammatory and cytotoxic potential of new phenanthrenoids from Luzula sylvatica, Molecules, 2020, 25 (10), 2372).
- purifier la fraction contenant les kinkeloides.

Cette méthode a permis de déterminer la quantité de kinkéloïdes dans la préparation méthanolique : entre 40 et 140 mg de kinkéloïdes par gramme d'une préparation méthanolique.

### 2. Writhing tests

Le test de crampe est une méthode chimique utilisée pour induire une douleur d'origine périphérique par l'injection de principes irritants tels que l'acide acétique chez la souris. L'activité analgésique du composé testé est déduite de la diminution de la fréquence des crampes.

L'administration par voie orale de la préparation méthanolique selon l'invention, en prise unique à la dose de 300 mg/kg, 30 min avant l'injection d'acide acétique, a réduit le nombre de crampes abdominales. Ce résultat confirme l'effet analgésique de cette préparation.

### 3. AlgoGram (marque déposée)

L'Algogram est un modèle de screening proposé par ANS biotech pour évaluer l'activité analgésique de molécules pharmaceutiques. Il met en jeu 11 modèles animaux de douleurs permettant de couvrir différents domaines de douleurs. Il est utile pour prédire sur quel type de douleur une molécule/préparation peut être actif.

Les résultats montrent que la préparation méthanolique, administré *peros* à 300 mg/kg 30 minutes avant l'évaluation de la douleur, confère un effet intéressant sur les douleurs viscérales, post-opératoires, inflammatoires et dans un modèle de douleur neuropathique (hypersensibilité au froid induite par l'oxaliplatine).

### 4. Activité anti-inflammatoire in vitro

Pour investiguer les mécanismes impliqués dans les effets analgésiques, l'évaluation des effets anti-inflammatoires de la préparation méthanolique à différentes concentrations a été réalisées dans un modèle *in vitro* de macrophages polarisés pro-inflammatoires. La préparation de plante a été testé à 4 concentrations, et ses effets ont été évalués par tests ELISA pour mesurer la production/diminution de cytokines pro-inflammatoires sur l'IL-1β, IL-6 et TNF-α, 3 cytokines majeures impliquées dans les réponses inflammatoires.

Il est observé que la production en TNF-α était significativement amoindrie en présence de la plus petite concentration en préparation de plante, montrant un effet anti-inflammatoire de la préparation de plante.

### 5. Conclusion pour la préparation méthanolique

Au global, une activité analgésique de la préparation méthanolique selon l'invention administré par voie orale en prise unique (300 mg/Kg) a pu être mise en évidence dans deux modèles de screening différents : le Writhing test (3 séries) et l'AlgoGram^{™}. Ce dernier montre un potentiel d'effet dans différents types de douleur : douleur viscérale, post-opératoire, inflammatoires et neuropathiques. Les données obtenues *in vitro* sur les macrophages pro-inflammatoires montrent que la préparation méthanolique selon l'invention pourrait exercer ses effets au moins en partie par une inhibition de la production de TNF-alpha.

### Exemple 2 - procédé d'extraction selon le deuxième mode de réalisation

### 1. Protocole d'extraction

- mélange de plante et d'eau, chauffé 30mn à 80°C ;
- Concentration de la préparation sous vide ;
- Pulvérisation de la préparation sur des feuilles de *Combretum* broyées.

### 2. AlgoGram

La présente préparation présente une inhibition de la douleur viscérale et de la douleur liée à une arthrite induite par du kaolin.

### 3. Conclusion

La présente préparation permet de conserver une partie de l'activité analgésique mise en évidence pour la préparation méthanolique.

### Exemple 3 - procédé d'extraction selon le troisième mode de réalisation

### 1. Protocole

- une étape de trempage d'une matière végétale broyée, en présence d'éthanol, avantageusement chauffé,
- une étape de percolation par l'ajout d'éthanol,
- une étape de retrempage par ajout d'éthanol,
- une étape de filtration du solvant de retrempage,
- une étape de concentration du filtrat et lyophilisation, par exemple par évaporation.

### 2. Writhing tests

Dans le Writhing test, la préparation selon l'invention présente une activité analgésique comparable à celle de la préparation méthanolique avec une réduction du nombre de crampes abdominales.

### 3. Algogram

La préparation selon l'invention administré par voie orale en prise unique à la dose de 300 mg/Kg présente dans l'AlgoGram un profil antalgique similaire à celui de la préparation méthanolique avec une amplitude d'effet plus faible.

L'inhibition est observée dans le modèle de douleur viscérale (TNBS) et dans les deux modèles de douleur inflammatoire (Kaolin et carraghénane, respectivement).

### 4. Conclusion

Une activité analgésique de la préparation selon l'invention, administré par voie orale en prise unique à la dose de 300 mg/kg, a pu être mise en évidence dans deux modèles de screening différents.

Le profil antalgique mis en évidence par ce dernier est similaire à celui de la préparation méthanolique bien que l'amplitude d'effet soit plus faible.

### Exemple 4 - procédé d'extraction selon le quatrième mode de réalisation

### 1. Protocole

- une étape de trempage de la matière végétale broyée, en présence d'éthanol,
- une étape de percolation par l'ajout d'éthanol,
- une étape de retrempage par ajout d'éthanol,
- une étape de filtration du solvant de retrempage,
- une étape de concentration du filtrat par évaporation,
- une étape de stabilisation de la préparation sec à l'alcool,
- une étape de concentration de la préparation végétal, et
- une étape de stabilisation à la glycérine de la préparation.

### 2. AlgoGram

La préparation selon l'invention, administré par voie orale en prise unique à la dose de 2000 mg/Kg, présente un profil antalgique à l'AlgoGram similaire à celui de la préparation méthanolique avec une amplitude d'effet moins importante.

Une inhibition de la douleur viscérale (modèle TNBS), et de la douleur inflammatoire (modèle au Kaolin et carraghénane), a pu être mise en évidence.

## Revendications

1. Préparation de *Combretum micranthum* G. Don, pour son utilisation chez des sujets humains ou animaux, dans le traitement et/ou la prévention de la douleur.

2. Préparation de *Combretum micranthum* G. Don, selon la revendication 1, **caractérisée en ce que** ladite douleur est choisie parmi les douleurs articulaires, viscérales, pelviennes, post-opératoires, inflammatoires, neuropathiques, arthritiques.

3. Préparation de *Combretum micranthum* G. Don, selon l'une quelconque des revendications 1 ou 2, **caractérisée** en que le traitement et/ou la prévention de la douleur est obtenu par l'intermédiaire d'un effet anti-inflammatoire.

4. Préparation de *Combretum micranthum* G. Don, selon l'une quelconque des revendications 1 à 3, **caractérisée** en que le traitement et/ou la prévention de la douleur englobe une diminution de son intensité et/ou de sa durée.

5. Préparation de *Combretum micranthum* G. Don, selon l'une quelconque des revendications 1 à 4, **caractérisée** en que la douleur est choisie parmi :
- les douleurs légères,
- les douleurs modérées, et
- les douleurs sévères.

6. Préparation de *Combretum micranthum* G. Don, selon l'une quelconque des revendications 1 à 5, **caractérisée** en que ladite préparation est issue d'une extraction à partir des feuilles.

7. Préparation de *Combretum micranthum* G. Don, selon l'une quelconque des revendications 1 à 6, **caractérisée en ce que** ladite préparation est issue d'un procédé choisi parmi les procédés suivants :
- un procédé d'extraction méthanolique comprenant les étapes successives suivantes :
-- une étape de broyage du matériel végétal,
-- une étape de macération du matériel végétal broyé dans un alcool, par exemple du méthanol,
-- une étape de filtration des macérats,
-- une étape de rassemblement des filtrats et évaporation pour éliminer l'alcool et l'eau,
-- une étape de lyophilisation ;
- un procédé de préparation d'une préparation végétale, à partir de matière végétale, comprenant :
-- une étape de contact entre la matière végétale et un solvant,
-- au moins une étape d'extraction en phase liquide des principes actifs, réalisée sous chauffage et sous pression réduite, pour obtenir une fraction d'extraction contenant lesdits principes actifs et une matière végétale résiduelle, et
-- une étape de séchage de ladite matière végétale résiduelle en présence de ladite fraction d'extraction, afin de permettre la fixation des principes actifs sur ladite matière végétale résiduelle,
- un procédé de préparation d'une préparation végétale comprenant :
-- une étape de trempage d'une matière végétale broyée, en présence d'un solvant alcoolique, par exemple de l'éthanol,
-- une étape de percolation par l'ajout d'un solvant alcoolique, par exemple de l'éthanol,
-- une étape de retrempage par ajout d'un solvant alcoolique, par exemple de l'éthanol,
-- une étape de filtration du solvant de retrempage,
-- une étape de concentration du filtrat et lyophilisation,
- un procédé de préparation d'une préparation végétale comprenant :
-- une étape de trempage de la matière végétale broyée, en présence d'un solvant alcoolique, par exemple de l'éthanol,
-- une étape de percolation par l'ajout d'un solvant alcoolique, par exemple de l'éthanol,
-- une étape de retrempage par ajout d'un solvant alcoolique, par exemple de l'éthanol,
-- une étape de filtration du solvant de retrempage,
-- une étape de concentration du filtrat par évaporation,
-- une étape de stabilisation de la préparation sec à l'alcool,
-- une étape de concentration de la préparation végétal, et
-- une étape de stabilisation à la glycérine de la préparation.

8. Préparation de *Combretum micranthum* G. Don, selon l'une quelconque des revendications 1 à 7, **caractérisée** en que ladite préparation contient des kinkeloïdes, avantageusement choisis parmi les kinkeloïdes A, les kinkeloïdes B, les kinkeloïdes C et les kinkeloïdes D.

9. Composition pharmaceutique ou non-pharmaceutique, contenant une préparation de *Combretum micranthum* G. Don selon l'une quelconque des revendications 1 à 8, pour son utilisation chez des sujets humains ou animaux, dans le traitement et/ou la prévention de la douleur.

10. Composition selon la revendication 9, contenant en outre l'un au moins des ingrédients suivants : une préparation d'algue, une préparation de plante, des probiotiques, des vitamines, des minéraux ou tout autre ingrédient nutritionnel.

11. Composition selon l'une quelconque des revendications 9 ou 10, adaptée à une utilisation dans l'industrie pharmaceutique, cosmétologique, agro-alimentaire, de la nutrition, des compléments alimentaires, de la nutrition animale.

12. Composition selon la revendication 11, adaptée à une utilisation dans l'industrie cosmétologique, agro-alimentaire, de la nutrition, des compléments alimentaires, de la nutrition animale, dans une utilisation non-thérapeutique d'une préparation de *Combretum micranthum* G. Don, pour son utilisation chez des sujets humains ou animaux, dans le traitement et/ou la prévention de la douleur.
